# EUROPÄISCHE PATENTANMELDUNG

(11) **EP 2 679 679 A1**
(43) Veröffentlichungstag der Anmeldung: **01.01.2014**
(21) Anmeldenummer: 13162829.9
(22) Anmeldetag: 09.04.2013
(51) Int. Cl.: C12N 9/96, C11D 3/386

(54) **Verwendung von Sulfopolymer als Enzymstabilisator**

(30) Priorität: 27.06.2012 DE 102012210992
(71) Anmelder: Henkel AG&Co. KGAA, 40589 Düsseldorf (DE)
(72) Erfinder: Mußmann, Nina, 47877 Willich (DE); Eiting, Thomas, 40589 Düsseldorf (DE); Bastigkeit, Thorsten, 42279 Wuppertal (DE); Benda, Konstantin, 40217 Düsseldorf (DE); Weber, Thomas, 41541 Dormagen (DE)

(57) **Zusammenfassung**

In wässerigen Systemen, insbesondere in maschinellen flüssigen Geschirrspülmitteln oder einer Komponente hierfür, wird eine höhere Stabilität von Enzymen, insbesondere von Amylasen und/oder Proteasen, durch die Verwendung von Sulfopolymer erreicht.

SEQUENCE LISTING

<110>  Henkel AG & Co. KGaA

<120>  Verwendung von Sulfopolymer als Enzymstabilisator

<130>  PT 019408 EP

<150>  DE 1020012210992.7
<151>  2012-06-27

<160>  2

<170>  PatentIn version 3.5

<210>  1
<211>  269
<212>  PRT
<213>  Bacillus sp.

<400>  1

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Asp Gly Glu Gly Ala Ile Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Ser Ser Ile Ser
145                 150                 155                 160

Tyr Pro Ala Arg Tyr Ala Asn Ala Met Ala Val Gly Ala Thr Asp Gln

                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210> 2
<211> 485
<212> PRT
<213> Bacillus sp.

<400> 2

His His Asn Gly Thr Asn Gly Thr Met Met Gln Tyr Phe Glu Trp Tyr
1               5                   10                  15

Leu Pro Asn Asp Gly Asn His Trp Asn Arg Leu Arg Ser Asp Ala Ser
            20                  25                  30

Asn Leu Lys Asp Lys Gly Ile Ser Ala Val Trp Ile Pro Pro Ala Trp
        35                  40                  45

Lys Gly Ala Ser Gln Asn Asp Val Gly Tyr Gly Ala Tyr Asp Leu Tyr
    50                  55                  60

Asp Leu Gly Glu Phe Asn Gln Lys Gly Thr Ile Arg Thr Lys Tyr Gly
65                  70                  75                  80

Thr Arg Asn Gln Leu Gln Ala Ala Val Asn Ala Leu Lys Ser Asn Gly
                85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn

115                 120                 125

Gln Glu Val Ser Gly Glu Tyr Thr Ile Glu Ala Trp Thr Lys Phe Asp
    130                 135                 140

Phe Pro Gly Arg Gly Asn Thr His Ser Asn Phe Lys Trp Arg Trp Tyr
145                 150                 155                 160

His Phe Asp Gly Val Asp Trp Asp Gln Ser Arg Lys Leu Asn Asn Arg
                165                 170                 175

Ile Tyr Lys Phe Arg Gly Asp Gly Lys Gly Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
                245                 250                 255

Thr Gly Lys Asn Met Phe Ala Val Ala Glu Phe Trp Lys Asn Asp Leu
            260                 265                 270

Gly Ala Ile Glu Asn Tyr Leu Asn Lys Thr Asn Trp Asn His Ser Val
        275                 280                 285

Phe Asp Val Pro Leu His Tyr Asn Leu Tyr Asn Ala Ser Lys Ser Gly
    290                 295                 300

Gly Asn Tyr Asp Met Arg Gln Ile Phe Asn Gly Thr Val Val Gln Arg
305                 310                 315                 320

His Pro Met His Ala Val Thr Phe Val Asp Asn His Asp Ser Gln Pro
                325                 330                 335

Glu Glu Ala Leu Glu Ser Phe Val Glu Glu Trp Phe Lys Pro Leu Ala
            340                 345                 350

Tyr Ala Leu Thr Leu Thr Arg Glu Gln Gly Tyr Pro Ser Val Phe Tyr
        355                 360                 365

Gly Asp Tyr Tyr Gly Ile Pro Thr His Gly Val Pro Ala Met Lys Ser
    370                 375                 380

Lys Ile Asp Pro Ile Leu Glu Ala Arg Gln Lys Tyr Ala Tyr Gly Arg
385                 390                 395                 400

Gln Asn Asp Tyr Leu Asp His His Asn Ile Ile Gly Trp Thr Arg Glu
                405                 410                 415

Gly Asn Thr Ala His Pro Asn Ser Gly Leu Ala Thr Ile Met Ser Asp
            420                 425                 430

Gly Ala Gly Gly Asn Lys Trp Met Phe Val Gly Arg Asn Lys Ala Gly
        435                 440                 445

Gln Val Trp Thr Asp Ile Thr Gly Asn Arg Ala Gly Thr Val Thr Ile
    450                 455                 460

Asn Ala Asp Gly Trp Gly Asn Phe Ser Val Asn Gly Gly Ser Val Ser
465                 470                 475                 480

Ile Trp Val Asn Lys
                485

## Beschreibung

Die vorliegende Erfindung betrifft die Verwendung von bestimmten Polymeren zur Stabilisierung bestimmter Enzyme sowie Kombinationsprodukte zum Waschen oder Reinigen, vorzugsweise zur Reinigung von harten Oberflächen, welche diese Polymere und Enzyme enthalten. Insbesondere betrifft diese Erfindung Kombinationsprodukte für die maschinelle Geschirreinigung sowie ein Verfahren zu ihrer Anwendung.

Waschmittel und Reinigungsmittel für harte Oberflächen wie auch Geschirrspülmittel stehen dem Verbraucher in einer Vielzahl von Angebotsformen zur Verfügung. Neben den traditionellen festen Mitteln gewinnen in letzter Zeit zunehmend fließfähige und insbesondere flüssige bis gelförmige Wasch- oder Reinigungsmittel an Bedeutung. Um die Reinigungsleistung fließfähiger Wasch- oder Reinigungsmittel zu verbessern, wurde im Stand der Technik nicht nur vorgeschlagen, Enzyme einzusetzen, sondern es werden auch Kombinationsprodukte beschrieben, welche ein Verpackungsmittel und zwei in diesem Verpackungsmittel befindliche voneinander getrennte fließfähige Wasch- oder Reinigungsmittel aufweisen. So offenbart die internationale Patentanmeldung WO 2007/025665 ein Verpackungsmittel und zwei in diesem Verpackungsmittel befindliche voneinander getrennte, fließfähige Wasch- oder Reinigungsmittel A und B, wobei die Zusammensetzung A Enzyme und die Zusammensetzung B keine Enzyme oder zumindest wenig Enzym aufweist. Die räumliche Trennung dieser beiden Zusammensetzungen macht es möglich, weitere Inhaltsstoffe und Charakteristika von Wasch- oder Reinigungsmitteln, welche die Stabilität der Enzyme beeinträchtigen könnten, von diesen Enzymen zu trennen. Inn der Regel weisen daher die enzymhaltige Zusammensetzung A und die nicht-enzymhaltige oder nur in geringen Mengen enzymhaltige Zusammensetzung B unterschiedliche pH-Werte auf. Hohe pH-Werte von beispielsweise 10 oder 11 oder sogar höher destabilisieren oder inaktivieren Enzyme, sind aber für die Reinigung hartnäckiger Anschmutzungen von Vorteil. In der genannten Patentanmeldung werden auch Beispiele genannt, welche ein so genanntes Sulfopolymer (Sulfosäuregruppen-haltiges Polymer) enthalten kann. In der Regel wird das Sulfopolymer als Bestandteil der Zusammensetzung B beschrieben; es gibt allerdings auch Beispiele, in denen auch die Zusammensetzungen A Sulfopolymer enthalten. Die Anmeldung offenbart nicht, dass Sulfopolymere Einfluss nehmen können auf die Stabilität von Enzymen, noch auf welche Enzyme Sulfopolymere stabilisierend wirken können.

Überraschenderweise wurde nun gefunden, dass die Stabilität von Proteasen und Amylasen in wässerigen Systemen durch das gleichzeitige Vorhandensein von Sulfopolymer verbessert werden kann.

Ein erster Gegenstand der Erfindung betrifft daher die Verwendung von Sulfopolymer in wässerigen Systemen, insbesondere in flüssigen maschinellen Geschirrspülmitteln oder Komponenten hierzu, zur Stabilisierung von Proteasen und/oder Amylasen. Vorzugsweise werden 0,1 bis 15 Gew.-% Sulfopolymer und 0,1 bis 10 Gew.-% einer Enzympräparation, enthaltend Amylase- und/oder Protease eingesetzt, wobei die Konzentrationen auf das wässerige System, insbesondere auf flüssige maschinelle Geschirrspülmittel oder eine Komponente hierfür, bezogen sind. Der pH-Wert dieser wässerigen Systeme, insbesondere der maschinellen Geschirrspülmittel oder der entsprechenden enzymhaltigen Komponente hierfür, beträgt vorzugsweise 6 bis 9 und insbesondere 6,5 bis 8,5.

Als Proteasen sind alle bekannten Proteasen des Standes der Technik geeignet. Unter ihnen sind solche vom Subtilisin-Typ bevorzugt. Beispiele hierfür sind die Subtilisine BPN' und Carlsberg sowie deren weiterentwickelte Formen, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7.

Eine bevorzugte Protease umfasst eine Aminosäuresequenz, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) oder die Aminosäure Asparagin (N) oder Glutamin (Q) oder die Aminosäure Alanin (A) oder Glycin (G) oder Serin (S) aufweist. Zunehmend bevorzugt ist die Aminosäuresequenz zu mindestens 81%, 82%, 83%, 84%, 85%, 86%, 87%, 88%, 89%, 90%, 91%, 92%, 93%, 94%, 95%, 96%, 97%, 98% und ganz besonders bevorzugt zu 99% identisch zu der in SEQ ID NO. 1 angegebenen Aminosäuresequenz. SEQ ID NO. 1 ist die Sequenz der reifen (maturen) alkalischen Protease aus Bacillus lentus DSM 5483, die offenbart ist in der internationalen Patentanmeldung WO 92/21760, und auf deren Offenbarung hiermit ausdrücklich Bezug genommen wird.

In einer weiteren Ausführungsform weist die Protease ferner in der Zählung gemäß SEQ ID NO. 1 mindestens eine der folgenden Aminosäuren aufweist:
(a) Threonin an Position 3 (3T),
(b) Isoleucin an Position 4 (4I),
(c) Alanin, Threonin oder Arginin an Position 61 (61A, 61T oder 61 R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (199I),
(h) Asparaginsäure, Glutaminsäure oder Glycin an Position 211 (211 D, 211 E oder 211 G),
(i) Kombinationen der Aminosäuren (a) bis (h).

Neben einer der genannten Aminosäuren an Position 99 weist die Protease daher eine oder mehrere der vorstehend genannten Aminosäuren an den jeweiligen Positionen auf. Diese Aminosäuren können weitere vorteilhafte Eigenschaften bewirken und/oder bereits vorhandene Eigenschaften noch verstärken. Insbesondere bewirken die vorstehend genannten Aminosäuren eine Steigerung der proteolytischen Aktivität und/oder der Stabilität der Protease in einem flüssigen Wasch- oder Reinigungsmittel bzw. in der durch dieses Wasch- oder Reinigungsmittel gebildeten Waschflotte.

Die Aminosäurepositionen werden hierbei durch ein Alignment der Aminosäuresequenz der einzusetzenden Protease mit der Aminosäuresequenz der Protease aus Bacillus lentus, wie sie in SEQ ID NO. 1 angegeben ist, definiert. Da die Protease aus Bacillus lentus im Stand der Technik ein wichtiges Referenzmolekül zur Beschreibung von Proteasen und von Aminosäureveränderungen darstellt ist es vorteilhaft, in der Zuordnung der Aminosäurepositionen auf die Zählung der Protease aus Bacillus lentus (SEQ ID NO. 1) Bezug zu nehmen. Weiterhin richtet sich die Zählung nach dem reifen (maturen) Protein. Diese Zuordnung ist insbesondere auch anzuwenden, wenn die Aminosäuresequenz der einzusetzenden Protease eine höhere Zahl von Aminosäurenresten umfasst als die Protease aus Bacillus lentus gemäß SEQ ID NO. 1. Ausgehend von den genannten Positionen in der Aminosäuresequenz der Protease aus Bacillus lentus sind die Aminosäurepositionen in einer erfindungsgemäß einzusetzenden Protease diejenigen, die eben diesen Positionen in einem Alignment zugeordnet sind.

Neben Position 99 besonders vorteilhafte Positionen sind demnach die Positionen 3, 4, 61, 154, 188, 193, 199 und 211, zuzuordnen in einem Alignment mit SEQ ID NO. 1 und damit in der Zählung gemäß SEQ ID NO. 1. In den genannten Positionen liegen in dem Wildtypmolekül der Protease aus Bacillus lentus folgende Aminosäurereste: S3, V4, G61, S154, A188, V193, V199, und L211. Besonders bevorzugt sind die Aminosäuren 3T, 4I, 61A, 154D, 154E, 211D, 211G und 211E, sofern die entsprechenden Positionen in einer erfindungsgemäß einzusetzenden Protease nicht schon natürlicherweise von einer dieser bevorzugten Aminosäuren eingenommen werden. Die Austausche 3T und 4I führen beispielsweise über einen Stabilisierungseffekt auf das Molekül zu einer Verbesserung der Lagerstabilität und der Reinigungsleistung der Protease und damit zu einer verbesserten Reinigungsleistung eines Phosphonat-haltigen flüssigen Wasch- oder Reinigungsmittels, das die Protease enthält.

Auf Grund der vorstehend genannten, für die jeweilige Position vorgesehenen Aminosäuren können sich weitere Sequenzabweichungen von SEQ ID NO. 1 ergeben, sofern SEQ ID NO. 1 eine andere Aminosäure in der jeweiligen Position aufweist. In Abhängigkeit von der Anzahl der vorliegenden Sequenzabweichungen von SEQ ID NO.1 ergeben sich daher unterschiedliche maximale Identitätswerte, die eine erfindungsgemäß einzusetzende Protease zu SEQ ID NO. 1 aufweisen kann, selbst wenn sie in allen übrigen Aminosäuren mit SEQ ID NO. 1 übereinstimmen sollte. Dieser Sachverhalt ist für jede mögliche Kombination der vorgeschlagenen Aminosäuren im Einzelfall zu berücksichtigen und ist ferner auch abhängig von der Länge der Aminosäuresequenz der Protease. Beispielsweise beträgt die maximale Identität bei einer, zwei, drei, vier, fünf, sechs, sieben, acht oder neun Sequenzveränderungen 99,63%, 99,26%, 98,88%, 98,51%, 98,14%, 97,77%, 97,40%, 97,03% bzw. 96,65% bei einer 269 Aminosäuren langen Aminosäuresequenz, beziehungsweise 99,64%, 99,27%, 98,91%, 98,55%, 98,18%, 97,82%, 97,45%, 97,09% bzw. 96,73% bei einer 275 Aminosäuren langen Aminosäuresequenz.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F.,Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Häufig genutzt werden beispielsweise Clustal (vgl. beispielsweise Chenna et al. (2003): Multiple sequence alignment with the Clustal series of programs. Nucleic Acid Research 31, 3497-3500) oder T-Coffee (vgl. beispielsweise Notredame et al. (2000): T-Coffee: A novel method for multiple sequence alignments. J. Mol. Biol. 302, 205-217) sowie BLAST oder FASTA für die Datenbanksuche, beziehungsweise Programme, die auf diesen Programmen bzw. Algorithmen basieren. Im Rahmen der vorliegenden Erfindung werden Sequenzvergleiche und Alignments bevorzugt mit dem Computer-Programm Vector NTI® Suite 10.3 (Invitrogen Corporation, 1600 Faraday Avenue, Carlsbad, Kalifornien, USA) mit den vorgegebenen Default-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

Für Amylasen können synonyme Begriffe verwendet werden, beispielsweise 1,4-alpha-D-Glucan-Glucanohydrolase oder Glycogenase. Erfindungsgemäß konfektionierbare Amylasen sind vorzugsweise α-Amylasen. Entscheidend dafür, ob ein Enzym eine α-Amylase im Sinne der Erfindung ist, ist deren Fähigkeit zur Hydrolyse von α(1-4)-Glykosidbindungen in der Amylose der Stärke.

Erfindungsgemäß einsetzbare Amylasen sind beispielsweise die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl® und von dem Unternehmen Danisco/Genencor unter dem Namen Purastar®ST erhältlich. Weiterentwicklungsprodukte dieser α-Amylase sind von dem Unternehmen Novozymes unter den Handelsnamen Duramyl® und Termamyl®ultra, von dem Unternehmen Danisco/Genencor unter dem Namen Purastar®OxAm und von dem Unternehmen Daiwa Seiko Inc., Tokyo, Japan, als Keistase® erhältlich. Die α-Amylase von Bacillus amyloliquefaciens wird von dem Unternehmen Novozymes unter dem Namen BAN® vertrieben, und abgeleitete Varianten von der α-Amylase aus Bacillus stearothermophilus unter den Namen BSG® und Novamyl®, ebenfalls von dem Unternehmen Novozymes. Des Weiteren sind für diesen Zweck die α-Amylase aus Bacillus sp. A 7-7 (DSM 12368) und die Cyclodextrin-Glucanotransferase (CGTase) aus Bacillus agaradherens (DSM 9948) hervorzuheben. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT® und Stainzyme® oder Stainzyme ultra® bzw. Stainzyme plus®, letztere ebenfalls von dem Unternehmen Novozymes. Auch durch Punktmutationen erhältliche Varianten dieser Enzyme können erfindungsgemäß eingesetzt werden. Besonders bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird bzw. deren diesbezüglicher Offenbarungsgehalt daher ausdrücklich in die vorliegende Patentanmeldung mit einbezogen wird.

Besonders geeignet für den Einsatz in erfindungsgemäßen Mitteln sind α-Amylase-Varianten der α-Amylase AA560 gemäß SEQ ID NO. 2. Folgende Varianten sind besonders vorteilhaft:
(a) α-Amylase-Variante, die gegenüber der α-Amylase AA560 gemäß SEQ ID NO. 2 eine, zwei, drei, vier, fünf oder sechs der folgenden Sequenzveränderungen in der Zählung der α-Amylase AA560 aufweist: R118K, D183* (Deletion), G184* (Deletion), N195F, R320K, R458K. Besonders bevorzugt weist die α-Amylase-Variante alle sechs der genannten Sequenzveränderungen auf.
(b) α-Amylase-Variante, die gegenüber der α-Amylase AA560 gemäß SEQ ID NO. 2 folgende Sequenzveränderungen aufweist (in der Zählung der α-Amylase AA560):
   (1) M9L/M202I,
   (2) M9L / M202I / M323T,
   (3) M9L / M202I / M323T / M382Y,
   (4) M9L / M202I / Y295F A339S,
   (5) M9L / M202I / Y295F,
   (6) M9L / M202I / A339S,
   (7) M9L / M202I / Y295F / A339S,
   (8) M9L / M202I / Y295F / A339S / E345R,
   (9) M9L / G149A / M202I / Y295F / A339S / E345R,
   (10) M9L / M202L,
   (11) M9L / M202L / M323T,
   (12) M9L / M202L / M323T / M382Y,
   (13) M9L / M202L / Y295F / A339S,
   (14) M9L / M202L / Y295F,
   (15) M9L / M202L / A339S,
   (16) M9L / M202L / Y295F / A339S,
   (17) M9L / M202L / Y295F / A339S, E345R,
   (18) M9L / G149A / M202L / Y295F / A339S / E345R,
   (19) M9L / M202T,
   (20) M9L / M202T / M323T,
   (21) M9L / M202T / M323T / M382Y,
   (22) M9L / M202T / Y295F / A339S,
   (23) M9L / M202T / Y295F,
   (24) M9L / M202T / A339S,
   (25) M9L / M202T / Y295F / A339S,
   (26) M9L / M202T / Y295F / A339S / E345R,
   (27) M9L / G149A / M202T / Y295F / A339S / E345R,
   (28) M9L / G149A / M202I / V214T / Y295F / N299Y / M323T / A339S / E345R,
   (29) M9L / G149A / M202L / V214I / Y295F / M323T / A339S / E345R / M382Y,
   (30) M9L / G149A / G182T / G186A / M202I / V214I / Y295F / N299Y / M323T / A339S,
   (31) M9L / G149A / G182T / G186A / M202L / T257I / Y295F / N299Y / M323T / A339S / E345R,
   (32) M9L / G149A / M202L / V214T / Y295F / N299Y / M323T / A339S / E345R,
   (33) M9L / G149A / M202I / V214I / Y295F / M323T / A339S / E345R / M382Y,
   (34) M9L / G149A / G182T / G186A / M202L / V214I / Y295F / N299Y / M323T / A339S,
   (35) M9L / G149A / G182T / G186A / M202I / T257I / Y295F / N299Y / M323T / A339S /E345R,
   (36) M9L / G149A / M202I / V214T / Y295F / N299Y / M323T / A339S / E345R / N471E,
   (37) M9L / G149A / M202L / V214I / Y295F / M323T / A339S / E345R / M382Y / N471 E,
   (38) M9L / G149A / G182T / G186A / M202I / V214I / Y295F / N299Y / M323T / A339S / N471 E,
   (39) M9L / G149A / G182T / G186A / M202L / T257I / Y295F / N299Y / M323T / A339S / E345R / N471 E,
   (40) M202L / M105F / M208F,
   (41) G133E / M202L / Q361E,
   (42) G133E / M202L / R444E,
   (43) M202L / Y295F,
   (44) M202L / A339S,
   (45) M202L / M323T,
   (46) M202L / M323T / M309L,
   (47) M202L / M323T / M430I,
   (48) M202L / V214T / R444Y,
   (49) M202L / N283D / Q361E,
   (50) M202L / M382Y / K383R,
   (51) M202L / K446R / N484Q,
   (52) M202I / Y295F,
   (53) M202I / A339S,
   (54) M202I / M105F / M208F,
   (55) G133E / M202I / Q361E,
   (56) G133E / M202I / R444E,
   (57) M202I / M323T,
   (58) M202I / M323T / M309L,
   (59) M202I / M323T / M430I,
   (60) M202I / V214T / R444Y,
   (61) M202I / N283D / Q361E,
   (62) M202I / M382Y / K383R,
   (63) M202I / K446R / N484Q,
   (64) M202V / M105F / M208F,
   (65) G133E / M202V / Q361 E,
   (66) G133E / M202V / R444E,
   (67) M202V / M323T,
   (68) M202V / M323T / M309L,
   (69) M202V / M323T / M430I,
   (70) M202V / M323T / M9L,
   (71) M202V / V214T / R444Y,
   (72) M202V / N283D / Q361E,
   (73) M202V / M382Y / K383R,
   (74) M202V / K446R / N484Q,
   (75) M202T / M 105F / M208F,
   (76) G133E / M202T / Q361E,
   (77) G133E / M202T / R444E,
   (78) M202T / Y295F,
   (79) M202T / A339S,
   (80) M202T / M323T,
   (81) M202T / M323T / M309L,
   (82) M202T / M323T / M430I,
   (83) M202T / M323T / M9L,
   (84) M202T / V214T / R444Y,
   (85) M202T / N283D / Q361E,
   (86) M202T / A339S,
   (87) M202T / Y295F
   (88) M202T / N299F,Y,
   (89) M202T / M382Y / K383R oder
   (90) M202T / K446R / N484Q

Hierunter ganz besonders bevorzugt sind folgende α-Amylase-Varianten:
(10) M9L / M202L,
(28) M9L / G149A / M202I / V214T / Y295F / N299Y / M323T / A339S / E345R,
(31) M9L / G149A / G182T / G186A / M202L / T257I / Y295F / N299Y / M323T / A339S / E345R,
(35) M9L / G149A / G182T / G186A / M202I / T257I / Y295F / N299Y / M323T /
(38) M9L / G149A G182T/ G186A/ / M202I / V214I / Y295F / N299Y / M323T /
(39) M9L / G149A G182T/ G186A/ / M202L / T257I / Y295F / N299Y / M323T / A339S / E345R / N471 E,
(45) M202L / M323T,
(46) M202L / M323T / M309L,
(62) M202I / M382Y / K383R,
(68) M202V / M323T / M309L,
(73) M202V / M382Y / K383R
(82) M202T / M323T / M4301 oder
(84) M202T / V214T / R444Y

(c) α-Amylase-Variante gemäß (b), die zusätzlich alle sechs unter (a) genannten Sequenzveränderungen aufweist, hierunter ganz besonders bevorzugt Variante 31 mit den sechs unter (a) genannten Sequenzveränderungen.

Erfindungsgemäß ganz besonders bevorzugt ist die vorstehend unter (a) genannte α-Amylase-Variante sowie die unter (c) genannte α-Amylase-Variante 31 mit den sechs unter (a) genannten Sequenzveränderungen.

Die Enzyme können in jeder nach dem Stand der Technik etablierten Form, einer so genannten Enzympräparation, eingesetzt werden. Hierzu gehören beispielsweise die durch Granulation, Extrusion, Verkapselung oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt. Im Rahmen der vorliegenden Erfindung wird unter Enzym oder Enzymen jeweils eine entsprechende Enzympräparation verstanden, wenn nicht eindeutig gekennzeichnet ist, dass das aktive Protein gemeint ist.

Weiterhin ist es möglich, zwei oder mehrere Enzyme zusammen zu konfektionieren, so dass ein einzelnes Granulat oder eine einzelne Enzympräparationen mehrere Enzymaktivitäten aufweist.

Sulfopolymere im Rahmen der vorliegenden Erfindung stellen Copolymere dar, welche zwei, drei, vier oder mehr unterschiedliche Monomereinheiten aufweisen können. Bevorzugte copolymere Sulfopolymere enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, - CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettiger oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen.

Bei den Sulfonsäuregruppen-haltigen Monomeren sind solche der Formel

R⁵(R⁶)C=C(R⁷)-X-SO₃H

bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für -COOH oder -COOR⁴ steht, wobei R⁴ ein gesättigter oder ungesättigter, geradkettiger oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₃)-CH₂-.

Unter diesen Monomeren sind solche der Formeln

H₂C=CH-X-SO₃H

H₂C=C(CH₃)-X-SO₃H

HO₃S-X-(R⁶)C=C(R⁷)-X-SO₃H

Bevorzugt, in denen R⁶ und R⁷ unabhängig voneinander ausgewählt sind aus -H, -CH₃, -CH₂CH₃, - CH₂CH₂CH₃ und -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₃)-CH₂-.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze.

In den Polymeren können die Sulfonsäuregruppen ganz oder teilweise in neutralisierter Form vorliegen, d.h. dass das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Der Einsatz von teil- oder vollneutralisierten sulfonsäuregruppenhaltigen Copolymeren ist erfindungsgemäß bevorzugt.

Die Monomerenverteilung der erfindungsgemäß bevorzugt eingesetzten Copolymere beträgt bei Copolymeren, die nur Carbonsäuregruppen-haltige Monomere und Sulfonsäuregruppen-haltige Monomere enthalten, vorzugsweise jeweils 5 bis 95 Gew.-%, besonders bevorzugt beträgt der Anteil des Sulfonsäuregruppen-haltigen Monomers 50 bis 90 Gew.-% und der Anteil des Carbonsäuregruppen-haltigen Monomers 10 bis 50 Gew.-%, die Monomere sind hierbei vorzugsweise ausgewählt aus den zuvor genannten.

Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte maschinelle Geschirrspülmittel sind dadurch gekennzeichnet, dass die Copolymere Molmassen von 2000 bis 200.000 gmol⁻¹, vorzugsweise von 4000 bis 25.000 gmol⁻¹ und insbesondere von 5000 bis 15.000 gmol⁻¹ aufweisen.

In einer weiteren bevorzugten Ausführungsform umfassen die Copolymere neben Carboxylgruppen-haltigem Monomer und Sulfonsäuregruppen-haltigem Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer maschineller Geschirrspülmittel verbessert werden.

In einer weiteren bevorzugten Ausführungsform der Erfindung enthalten die Mittel mindestens ein anionisches Copolymer, umfassend
i) Carbonsäuregruppen-haltige Monomer(e)
ii) Sulfonsäuregruppen-haltige Monomer(e)
iii) nichtionische Monomer(e).

Als nichtionische Monomere werden vorzugsweise Monomere der allgemeinen Formel R¹(R²)C=C(R³)-X-R⁴ eingesetzt, in der R¹ bis R³ unabhängig voneinander für -H, -CH₃ oder -C₂H₅ steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Diemthylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C22-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2,Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, N-(Methyl)acrylamid, Acrylsäure-2-Ethylhexylester, Methacrylsäure-2-Ethylhexylester, N-(2-Ethylhexyl)acrylamid, Acrylsäureoctylester, Methacrylsäureoctylester, N-(Octyl)acrylamid, Acrylsäurelaurylester, Methacrylsäurelaurylester, N-(Lauryl)acrylamid, Acrylsäurestearylester, Methacrylsäurestearylester, N-(Stearyl)acrylamid, Acrylsäurebehenylester, Methacrylsäurebehenylester und N-(Behenyl)acrylamid oder deren Mischungen.

Ein weiterer Gegenstand der Erfindung betrifft ein Kombinationsprodukt, umfassend ein Verpackungsmittel und mindestens zwei in diesem Verpackungsmittel befindliche voneinander getrennte, wässerige Wasch- oder Reinigungskomponenten A und B, wobei A und B enthalten
A:
   - 10 bis 74,9 Gew.-% Gerüststoff(e);
   - 0,1 bis 10 Gew.-% Enzympräparation, enthaltend Amylase- und/oder Protease;
   - 24,9 bis 89,8 Gew.-% Wasser; und
B:
   - 10 bis 75 Gew.-% Gerüststoff(e);
   - weniger als 0,1 Gew.-% Enzympräparation;
   - 25 bis 90 Gew.-% Wasser;
wobei die Komponente A zusätzlich mindestens ein Sulfopolymer, vorzugsweise in Mengen von 0,01 bis 15 Gew.-%, bezogen auf die Komponente A, enthält. Enthält die Komponente A mehr als 0,1 Gew.-% Sulfopolymer, so geschieht dies im Austausch gegen die übrigen Komponenten, insbesondere im Austausch gegenüber Gerüststoffen und/oder Wasser, so dass die Höchstmengen an Gerüststoffen und/oder Wasser entsprechend reduziert werden.

In einer bevorzugten Ausführungsform der Erfindung enthält die Komponente A 1 bis 15 Gew.-%, insbesondere 1,5 bis 10 Gew.-% Sulfopolymer. Die Komponente B kann ebenfalls Sulfopolymer enthalten; der Gewichtsanteil des Sulfopolymers kann in der Komponente B sogar höher sein als in der Komponente A.

Insbesondere sind Kombinationsprodukte bevorzugt, in denen der Gewichtsanteil des Sulfopolymers in der Komponente A höher ist als der Gewichtsanteil des Sulfopolymers in der Komponente B oder nur die Komponente A Sulfopolymer enthält.

Phosphonate können als Komplexierungsmittel eingesetzt werden. Phosphonate können sowohl in der Zusammensetzung A als auch in der Zusammensetzung B oder nur in einer der beiden Zusammensetzungen enthalten sein. Überraschenderweise wurde jedoch festgestellt, dass auch Phosphonate einen Einfluss auf die Enzymstabilität haben können. Während Phosphonate Proteasen zu stabilisieren scheinen, können sie auf Amylasen einen destabilisierenden Effekt ausüben. In einer bevorzugten Ausführungsform enthalten die Zusammensetzungen A und B beide Phosphonate, wobei der Gehalt an Phosphonaten in der Zusammensetzung A, bezogen auf die Zusammensetzung A, niedriger ist als der Gehalt an Phosphonat in der Zusammensetzung B, bezogen auf die Zusammensetzung B. In einer weiteren bevorzugten Ausführungsform enthält die Zusammensetzung B Phosphonat, während die Zusammensetzung A frei von Phosphonaten ist.

Ein weiterer Gegenstand der Erfindung betrifft ein automatisches Wasch- oder Reinigungsverfahren, insbesondere ein maschinelles Geschirrspülverfahren, bei welchem aus einem Kombinationsprodukt, umfassend ein Verpackungsmittel und mindestens zwei in diesem Verpackungsmittel befindliche voneinander getrennte, wässerige Wasch- oder Reinigungskomponenten A und B, wobei A und B enthalten
A:
   - 10 bis 74,9 Gew.-% Gerüststoff(e);
   - 0,1 bis 10 Gew.-% Enzympräparation, enthaltend Amylase- und/oder Protease;
   - 24,9 bis 89,8 Gew.-% Wasser; und
B:
   - 10 bis 75 Gew.-% Gerüststoff(e);
   - weniger als 0,1 Gew.-% Enzympräparation;
   - 25 bis 90 Gew.-% Wasser;
und wobei ferner die Komponente A zusätzlich mindestens ein Sulfopolymer, vorzugsweise in Mengen von 0,01 bis 15 Gew.-%, bezogen auf die Komponente A, enthält,
- die Wasch- oder Reinigungskomponenten A und B aus einem wasserunlöslichen Verpackungsmittel in eine in der Maschine vorgesehene oder in eine separate Dosiervorrichtung oder direkt in die Maschine dosiert werden
   oder
- das wasserlösliche Verpackungsmittel in eine in der Maschine vorgesehene oder in eine separate Dosiervorrichtung dosiert oder direkt in den Innenraum der Maschine gelegt
   und anschließend der Wasch- oder Reinigungsvorgang in Gang gesetzt wird.

Werden in der Komponente A mehr als 0,1 Gew.-% Sulfopolymer eingesetzt, so geschieht dies im Austausch gegen die übrigen Komponenten, insbesondere im Austausch gegenüber Gerüststoffen und/oder Wasser, so dass die Höchstmengen an Gerüststoffen und/oder Wasser entsprechend reduziert werden.

Die erfindungsgemäßen Kombinationsprodukte umfassen neben den beiden flüssigen Reinigungskomponenten A und B weiterhin ein Verpackungsmittel. In diesem Verpackungsmittel liegen die beiden Reinigungskomponenten A und B voneinander getrennt vor, das heißt, sie bilden keine gemeinsame Phasengrenze aus, sondern befinden sich vielmehr in voneinander getrennten, beispielsweise durch eine Trennwand getrennten Bereichen des Verpackungsmittels.

In einer Ausführungsform der Erfindung ist das Verpackungsmittel als wasserlöslicher Zwei- oder Mehrkammerbehälter, beispielsweise als Zwei- oder Mehrkammerbeutel ausgestaltet.

Als ein solches Verpackungsmittel ist aber auch ein wasserunlöslicher Zwei- oder Mehrkammerbehälter geeignet. Ein solcher Zwei- oder Mehrkammerbehälter weist nicht zwingend, aber typischerweise ein Gesamtvolumen zwischen 100 und 5000 ml, vorzugsweise zwischen 200 und 2000 ml auf. Das Volumen der einzelnen Kammern beträgt vorzugsweise zwischen 50 und 2000 ml, bevorzugt zwischen 100 und 1000 ml. Bevorzugte Zwei- oder Mehrkammerbehälter weisen eine Flaschenform auf.

Zur Dosierung der wässerigen Wasch- oder Reinigungsmittel verfügt der wasserunlösliche Zwei- oder Mehrkammerbehälter vorzugsweise über mindestens einen Ausguss, der beispielsweise in Form eines gemeinsamen Ausgusses für alle in der Flasche enthaltenen Komponenten ausgestaltet sein kann. Bevorzugt werden jedoch solche Zwei- oder Mehrkammerbehälter, bei denen jede der Aufnahmekammern des Behälters über einen eigenen Ausguss verfügt. Durch eine solche Ausgestaltung wird beispielsweise eine Kontamination einzelner Kammern durch Inhaltsstoffe aus einer anderen Kammer vermieden.

Erfindungsgemäße Kombinationsprodukte, bei denen es sich bei dem Verpackungsmittel um einen wasserunlöslichen Zwei- oder Mehrkammerbehälter handelt, wobei vorzugsweise jede der Aufnahmekammern des Verpackungsmittels mit einem Ausguss versehen ist, werden bevorzugt.

Die Dosierung der beiden wässerigen Wasch- oder Reinigungskomponenten A und B kann im Falle eines Waschmittels beispielsweise über eine Dosierschublade der Waschmaschine oder über eine separate Dosiervorrichtung wie eine Dosierkugel, welche direkt in die Waschtrommel gelegt wird, erfolgen. Im Falle eines maschinellen Geschirrspülmittelprodukts kann die Dosierung beispielsweise in die Dosierkammer in der Tür oder einen zusätzlichen Dosierbehälter im Innenraum der Geschirrspülmaschine oder direkt auf das verschmutzte Geschirr erfolgen. Alternativ können die beiden Wasch- oder Reinigungskomponenten auch auf eine der Innenwände der Geschirrspülmaschine, beispielsweise die Innenseite der Tür, dosiert werden.

Wasserlöslichen Zwei- oder Mehrkammerbehälter werden vorzugsweise direkt in den Innenraum der Maschine oder insbesondere im Falle von maschinellen Geschirrspülverfahren in die eingebaute Dosiervorrichtung der Maschine gelegt.

Die wässerigen Wasch- oder Reinigungskomponenten A und B müssen unter Anwendungsbedingungen fließfähig sein. Darunter wird verstanden, dass die Komponenten A und B sich ohne weitere Hilfsmittel aus dem wasserunlöslichen Verpackungsmittel in die vorgesehene Maschine oder Dosiervorrichtung dosieren lassen. Sollte das wasserunlösliche Verpackungsmittel aus einem flexiblen Material bestehen, so können die Komponenten A und/oder B auch ein derart rheologisches Verhalten zeigen, dass sie im Ruhezustand in dem wasserunlöslichen Verpackungsmittel nicht fließfähig sind, sich aber durch leichten Druck des Anwenders auf das wasserunlösliche Verpackungsmittel oder durch Schütteln in einen fließfähigen Zustand überführen und somit dosieren lassen. In der Regel handelt es sich dabei um flüssige Mittel, die unter normalen Anwendungsbedingungen fließfähig sind und deren Viskositäten in einem breiten Rahmen variieren können. Im Rahmen der vorliegenden Erfindung zählen zu den flüssigen Komponenten auch gelförmige oder pastöse Mittel.

Bevorzugte Kombinationsprodukte der vorliegenden Erfindung umfassen fließfähige, insbesondere flüssige bis gelförmige Reinigungsmittel, welche sich für den Einsatz in maschinellen Geschirrspülmaschinen eignen.

Die in den erfindungsgemäßen Kombinationsprodukten getrennt voneinander vorliegenden flüssigen Reinigungskomponenten A und B enthalten zusätzlich zu den bereits genannten Inhaltsstoffen Gerüststoffe. Zu den Gerüststoffen zählen dabei insbesondere Zeolithe, Silikate, Carbonate, organische Cobuilder und - wo keine ökologischen Vorurteile gegen ihren Einsatz bestehen - auch die Phosphate.

Als Silikate kommen amorphe Natriumsilikate mit einem Modul Na₂O : SiO₂ von 1:2 bis 1:3,3, vorzugsweise von 1:2 bis 1:2,8 und insbesondere von 1:2 bis 1:2,6, aber auch kristalline schichtförmige Silikate der allgemeinen Formel NaMSiₓO₂ₓ₊₁ · y H₂O, worin M Natrium oder Wasserstoff darstellt, x eine Zahl von 1,9 bis 22, vorzugsweise von 1,9 bis 4, wobei besonders bevorzugte Werte für x 2, 3 oder 4 sind, und y für eine Zahl von 0 bis 33, vorzugsweise von 0 bis 20 steht, in Betracht.

Im Rahmen der vorliegenden Erfindung ist es bevorzugt, dass diese(s) Silikat(e), vorzugsweise Alkalisilikate, besonders bevorzugt kristalline oder amorphe Alkalidisilikate, in den flüssigen Reinigungsmittel A und/oder B in Mengen von 2 bis 40 Gew.-%, vorzugsweise von 3 bis 30 Gew.-% und insbesondere von 5 bis 25 Gew.-%, jeweils bezogen auf das Gewicht des jeweiligen Reinigungsmittels A oder B, enthalten sind.

Bis zum heutigen Tag werden Phosphate in maschinellen Geschirrspülmitteln in weiten Teilen der Welt immer noch bevorzugt eingesetzt ist. Unter der Vielzahl der kommerziell erhältlichen Phosphate haben die Alkalimetallphosphate unter besonderer Bevorzugung von Pentanatrium- bzw. Pentakaliumtriphosphat (Natrium- bzw. Kaliumtripolyphosphat) in der Wasch- und Reinigungsmittel-Industrie die größte Bedeutung.

Alkalimetallphosphate ist dabei die summarische Bezeichnung für die Alkalimetall- (insbesondere Natrium- und Kalium-) Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei.

Werden im Rahmen der vorliegenden Anmeldung Phosphate als wasch- oder reinigungsaktive Substanzen in den flüssigen Reinigungskomponenten A und/oder B eingesetzt, so enthalten bevorzugte Kombinationsprodukte diese(s) Phosphat(e), vorzugsweise Alkalimetallphosphat(e), besonders bevorzugt Pentanatrium- und/oder Pentakaliumtriphosphat (Natrium- und/oder Kaliumtripolyphosphat), in Mengen von 5 bis 60 Gew.-%, vorzugsweise von 15 bis 45 Gew.-% uns insbesondere von 20 bis 40 Gew.-%, jeweils bezogen auf das Gewicht der jeweiligen Reinigungskomponente A oder B. In einer bevorzugten Ausführungsform der Erfindung weist die Komponente A weniger Phosphate auf als die Komponente B. In einer weiteren bevorzugten Ausführungsform weist die Komponente A Phosphate in Mengen von 5 bis 15 Gew.-%, bezogen auf die Komponente A, und die Komponente B von 10 bis 20 Gew.-%, bezogen auf die Komponenten B, auf, mit der Maßgabe, dass die Phosphatmenge in Komponente B höher ist als in Komponente A.

Als organische Cobuilder sind insbesondere Polycarboxylate / Polycarbonsäuren, polymere Polycarboxylate, Asparaginsäure, Polyacetale, Dextrine, weitere organische Cobuilder sowie Phosphonate zu nennen.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen. Eine weitere bedeutende Klasse der phosphatfreien Gerüststoffe stellen Aminocarbonsäure und/oder ihre Salze dar. Besonders bevorzugte Vertreter dieser Klasse sind Methylglycindiessigsäure (MGDA) oder ihre Salze sowie Glutamindiessigsäure (GLDA) oder ihre Salze. Der Gehalt an diesen Aminocarbonsäuren bzw. ihren Salzen kann beispielsweise, bezogen auf die Summe der Mittel A und B, zwischen 0,1 und 15 Gew.-% ausmachen.

Als Gerüststoffe sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Als weiteren Bestandteil können die erfindungsgemäßen Kombinationsprodukte zur Steigerung der Wasch-, beziehungsweise Reinigungsleistung weitere Enzyme enthalten. Hierzu gehören insbesondere Lipasen, Cutinasen, Cellulasen, Hemicellulasen, zu denen insbesondere auch Mannanasen, Xanthanlyasen, Pektinlyasen (=Pektinasen), Pektinesterasen, Pektatlyasen, Xyloglucanasen (=Xylanasen), Pullulanasen und β-Glucanasen zählen, oder Oxidoreduktasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Wasch- oder Reinigungsmittel enthalten Enzyme vorzugsweise in Gesamtmengen von 1 x 10⁻⁶ bis 5 Gew.-%, bezogen auf aktives Protein. Die Proteinkonzentration kann mit Hilfe bekannter Methoden, zum Beispiel dem BCA-Verfahren oder dem Biuret-Verfahren bestimmt werden.

Wie eingangs ausgeführt, beträgt der Gewichtsanteil der Enzyme am Gesamtgewicht der fließfähigen Reinigungskomponente A 0,1 bis 10 Gew.-%, vorteilhafterweise 0,2 bis 9 Gew.-% und insbesondere 0,5 bis 8 Gew.-%. Besonders gute Reinigungsleistungen werden erzielt, wenn der Gewichtsanteil des Enzyms am Gesamtgewicht der Reinigungskomponente A 1 bis 5 Gew.-% beträgt.

Obgleich die fließfähige Reinigungskomponente B selbstverständlich auch Enzyme enthalten kann, ist es doch bevorzugt, dass der Enzymgehalt der Reinigungskomponente B möglichst gering ist und weniger als 0,1 Gew.-% beträgt. Besonders bevorzugte Kombinationsprodukte sind dadurch gekennzeichnet, dass die fließfähige Reinigungskomponente B keine Enzyme enthält und die gesamte Enzymmenge über die fließfähige Reinigungskomponente A zur Verfügung gestellt wird. Insbesondere wird die Gesamtmenge an Proteasen und/oder Amylasen, vorteilhafterweise an Proteasen und Amylasen, nur durch die Komponente A zur Verfügung gestellt.

Bevorzugt werden ein oder mehrere Enzyme und/oder Enzympräparationen, vorzugsweise feste oder flüssige Protease-Präparationen und/oder Amylase-Präparationen eingesetzt. In einer besonders bevorzugten Ausführungsform weist die flüssige Reinigungskomponente A eine Kombination von Protease- und Amylase-Präparationen auf, wobei die Mengen an Enzympräparationen in der Komponente A vorteilhafterweise zwischen 1 und 10 Gew.-%, bezogen auf die Komponente A, betragen.

Die erfindungsgemäßen Kombinationsprodukte können auch Enzymstabilisatoren beinhalten. Eine Gruppe von Stabilisatoren sind reversible Proteaseinhibitoren. Häufig werden hierfür Benzamidin-Hydrochlorid, Borax, Borsäuren, Boronsäuren oder deren Salze oder Ester eingesetzt, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2 bis 50 Monomeren werden zu diesem Zweck eingesetzt. Zu den peptidischen reversiblen Proteaseinhibitoren gehören unter anderem Ovomucoid und Leupeptin. Auch spezifische, reversible Peptid-Inhibitoren für die Protease Subtilisin sowie Fusionsproteine aus Proteasen und spezifischen Peptid-Inhibitoren sind hierfür geeignet.

Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise Bernsteinsäure, andere Dicarbonsäuren oder Salze der genannten Säuren. Auch endgruppenverschlossene Fettsäureamidalkoxylate sind für diesen Zweck geeignet. Bestimmte als Builder eingesetzte organische Säuren vermögen, wie in WO 97/18287 offenbart, zusätzlich ein enthaltenes Enzym zu stabilisieren.

Weitere Enzymstabilisatoren sind dem Fachmann aus dem Stand der Technik bekannt.

Besonders bevorzugt werden Kombinationen von Stabilisatoren eingesetzt, beispielsweise aus Polyolen, wie Glycerin, Ethylenglykol, Propylenglykol oder Sorbit, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren wird günstigerweise durch die Kombination mit Borsäure und/oder Borsäurederivaten und Polyolen gesteigert und noch weiter durch die zusätzliche Wirkung von zweiwertigen Kationen, wie zum Beispiel Calcium-Ionen.

Die zuvor beschriebenen erfindungsgemäßen Mittel können neben den zuvor beschriebenen Inhaltsstoffen weitere wasch- und reinigungsaktive Substanzen enthalten, vorzugsweise wasch- und reinigungsaktive Substanzen aus der Gruppe der Tenside, Polymere, Glaskorrosionsinhibitoren, Korrosionsinhibitoren, Alkaliträger, Duftstoffe und Parfümträger, Farbstoffen und Konservierungsmittel. Bleichmittel und Bleichaktivatoren, insbesondere flüssige Bleichaktivatoren können ebenfalls vorhanden sein. Diese bevorzugten Inhaltsstoffe werden in der Folge näher beschrieben.

Zur Gruppe der Tenside werden die nichtionischen, die anionischen, die kationischen und die amphoteren Tenside gezählt.

Zu den anionischen Tensiden zählen solche mit mindestens einer Sulfat- oder Sulfonatgruppe, vorzugsweise ausgewählt aus Fettalkoholsulfaten, Ethersulfaten, Alkansulfonaten und Alkylbenzolsulfonaten. Bevorzugt sind hierbei C₁₂-C₁₈-Fettalkohol-Sulfate, z.B. Sulfopon K 35 (BASF, Deutschland), ethoxylierte C₁₂-C₁₄-Ethersulfate, beispielsweise Texapon N70 (BASF, Deutschland), sekundäre C₁₃-C₁₇-Alkansulfonate, z.B. Hostapur SAS 93 (Clariant, Deutschland), sowie lineare C8-C18-Alkylbenzolsulfonate, insbesondere Dodecylbenzolsulfonat oder C₉-C₁₃-Alkylbenzolsulfonat.

Als nichtionische Tenside können alle dem Fachmann bekannten nichtionischen Tenside eingesetzt werden. Zu diesen zählen beispielsweise ethoxylierte und/oder propoxylierte Alkohole oder Fettalkohole wie auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide.

Niotenside aus der Gruppe der alkoxylierten Alkohole, insbesondere der alkoxylierten linearen C₈-C₁₈-Alkohole oder der methylverzweigten geradekettigen oder ungeradkettigen Alkohole, besonders bevorzugt aus der Gruppe der gemischt alkoxylierten Alkohole und insbesondere aus der Gruppe der EO-AO-EO-Niotenside, werden mit besonderem Vorzug eingesetzt.

Als besonders bevorzugte Niotenside haben sich im Rahmen der vorliegenden Erfindung schwachschäumende Niotenside erwiesen, welche alternierende Ethylenoxid- und Alkylenoxideinheiten aufweisen. Unter diesen sind wiederum Tenside mit EO-AO-EO-AO-Blöcken bevorzugt, wobei jeweils eine bis zehn EO- bzw. AO-Gruppen aneinander gebunden sind, bevor ein Block aus den jeweils anderen Gruppen folgt. Hier sind nichtionische Tenside der allgemeinen Formel 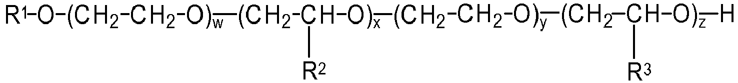 bevorzugt, in der R¹ für einen geradkettigen oder verzweigten, gesättigten oder ein- bzw. mehrfach ungesättigten C₆₋₂₄-Alkyl- oder -Alkenylrest steht; jede Gruppe R² bzw. R³ unabhängig voneinander ausgewählt ist aus -CH₃, -CH₂CH₃, -CH₂CH₂-CH₃, CH(CH₃)₂ und die Indizes w, x, y, z unabhängig voneinander für ganze Zahlen von 1 bis 6 stehen. Somit sind insbesondere nichtionische Tenside bevorzugt, die einen C₉₋₁₅-Alkylrest mit 1 bis 4 Ethylenoxideinheiten, gefolgt von 1 bis 4 Propylenoxideinheiten, gefolgt von 1 bis 4 Ethylenoxideinheiten, gefolgt von1 bis 4 Propylenoxideinheiten aufweisen. Diese Tenside weisen in wässriger Lösung die erforderliche niedrige Viskosität auf und sind erfindungsgemäß mit besonderem Vorzug einsetzbar.

Tenside der allgemeinen Formel

R¹-CH(OH)CH₂O-(AO)_{w}(A'O)ₓ(A"O)_{y}-(A"'O)_{z}R², in der

R¹ und R² unabhängig voneinander für einen geradkettigen oder verzweigten, gesättigten oder ein- bzw. mehrfach ungesättigten C₂₋₄₀-Alkyl- oder -Alkenylrest steht; A, A', A" und A'" unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) steht; und w, x, y und z für Werte zwischen 0,5 und 90 stehen, wobei x, y und/oder z auch 0 sein können, sind erfindungsgemäß besonders bevorzugt.

Ganz besonders bevorzugte nichtionische Tenside weisen in einer bevorzugten Ausführungsform die allgemeine Formel R¹O[CH₂CH(CH₃)O]ₓ[CH₂CH₂O]_{y}[CH₂CH(CH₃)O]_{z}CH₂CH(OH)R² auf, in der R¹ für einen linearen oder verzweigten aliphatischen Kohlenwasserstoffrest mit 4 bis 22 Kohlenstoffatomen oder Mischungen hieraus steht, R² einen linearen oder verzweigten Kohlenwasserstoffrest mit 2 bis 26 Kohlenstoffatomen, insbesondere 4 bis 20 Kohlenstoffatomen, oder Mischungen hieraus bezeichnet und x und z für Werte zwischen 0 und 40 und y für einen Wert von mindestens 15 steht.

Der Zusatz dieser nichtionischen Tenside hat sich insbesondere in Bezug auf die Klarspülleistung und die Trocknung als vorteilhaft erwiesen. In einer bevorzugten Ausführungsform enthält das maschinelle Geschirrspülmittel, bezogen auf die Summe der Komponenten A und B, nichtionisches Tensid der allgemeinen Formel R¹O[CH₂CH(CH₃)O]ₓ[CH₂CH₂O]_{y}[CH₂CH(CH₃)O]_{z}CH₂CH(OH)R² in Mengen von 0,1 bis 15 Gew.-%, vorzugsweise 0,2 bis 10 Gew.-%, besonders bevorzugt 0,5 bis 8 Gew.-% und insbesondere von 1,0 bis 6 Gew.-%.

Bevorzugt sind insbesondere solche endgruppenverschlossene poly(oxyalkylierten) Niotenside, die, gemäß der Formel R¹O[CH₂CH₂O]_{y}CH₂CH(OH)R² enthält, in der R¹ für einen linearen oder verzweigten aliphatischen Kohlenwasserstoffrest mit 4 bis 22, insbesondere 6 bis 16 Kohlenstoffatomen oder Mischungen hieraus steht, R² einen linearen oder verzweigten Kohlenwasserstoffrest mit 2 bis 26, insbesondere 4 bis 20 Kohlenstoffatomen oder Mischungen hieraus bezeichnet und y für einen Wert zwischen 15 und 120 vorzugsweise 20 bis 100, insbesondere 20 bis 80 steht. Zur Gruppe dieser nichtionischen Tenside zählen beispielsweise Hydroxymischether der allgemeinen Formel C₆₋₂₂-CH(OH)CH₂O-(EO)₂₀₋₁₂₀-C₂₋₂₆, zum Beispiel die C₈₋₁₂ Fettalkohol-(EO)₂₂-2-hydroxydecylether und die C₄₋₂₂ Fettalkohol-(EO)₄₀₋₈₀-2-hydroxyalkylether.

Als weiteres nichtionisches Tensid ist ein Tensid der allgemeinen Formel R¹CH(OH)CH₂O-(CH₂CH₂O)₂₀₋₁₂₀- R², wobei R¹ und R² unabhängig voneinander für einen linearen oder verzweigten aliphatischen Kohlenwasserstoffrest mit 2 bis 20 Kohlenstoffatomen stehen, besonders bevorzugt.

Bevorzugt sind weiterhin Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 22 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 4, vorzugsweise 0,5 bis 1,5, und y für einen Wert von mindestens 15 steht.

Auch Tenside der allgemeinen Formel R¹O[CH₂CH(CH₃)O]ₓ[CH₂CH₂O]_{y}CH₂CH(OH)R² sind bevorzugt, in der R¹ für einen linearen oder verzweigten aliphatischen Kohlenwasserstoffrest mit 4 bis 22 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für einen Wert zwischen 1 und 40 und y für einen Wert zwischen 15 und 40 steht, wobei die Alkyleneinheiten [CH₂CH(CH₃)O] und [CH₂CH₂O] randomisiert, d.h. in Form einer statistischen, zufälligen Verteilung vorliegen.

Zur Gruppe der bevorzugten endgruppenverschlossene poly(oxyalkylierten) Niotenside zählen auch Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel
R¹O[CH₂CH(R³)O]ₓ[CH₂]ₖCH(OH)[CH₂]ⱼOR²,
in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen.
Wenn der Wert x ≥ 2 ist, kann jedes R³ in der oben stehenden Formel
R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der oben stehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der oben stehenden Formel weisen Werte von k = 1 und j = 1 auf, so dass sich die vorstehende Formel zu

R¹O[CH₂CH(R³)O]ₓCH₂CH(OH)CH₂OR²

vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Weitere bevorzugt eingesetzte nichtionische Tenside sind nichtionische Tenside der allgemeinen Formel R¹O(AlkO)ₓM(OAlk)_{y}OR², wobei
R¹ und R² unabhängig voneinander für einen verzweigten oder unverzweigten, gesättigten oder ungesättigten, gegebenenfalls hydroxylierten Alkylrest mit 4 bis 22 Kohlenstoffatomen stehen;
Alk für einen verzweigten oder unverzweigten Alkylrest mit 2 bis 4 Kohlenstoffatomen steht;
x und y unabhängig voneinander für Werte zwischen 1 und 70 stehen; und
M für einen Alkylrest aus der Gruppe CH₂, CHR³, CR³R⁴, CH₂CHR³ und CHR³CHR⁴ steht, wobei R³ und R⁴ unabhängig voneinander für einen verzweigten oder unverzweigten, gesättigten oder ungesättigten Alkylrest mit 1 bis 18 Kohlenstoffatomen stehen.

Bevorzugt sind hierbei nichtionische Tenside der allgemeinen Formel
R¹-CH(OH)CH₂-O(CH₂CH₂O)ₓCH₂CHR(OCH₂CH₂)_{y}O-CH₂CH(OH)-R², wobei
- R, R¹ und R² unabhängig voneinander für einen Alkylrest oder Alkenylrest mit 6 bis 22 Kohlenstoffatomen;
- x und y unabhängig voneinander für Werte zwischen 1 und 40 stehen

Bevorzugt sind hierbei insbesondere Verbindungen der allgemeinen Formel R¹-CH(OH)CH₂-O(CH₂CH₂O)ₓCH₂CHR(OCH₂CH₂)_{y}O-CH₂CH(OH)-R², in denen R für einen linearen, gesättigten Alkylrest mit 8 bis 16 Kohlenstoffatomen, vorzugsweise 10 bis 14 Kohlenstoffatomen steht und n und m unabhängig voneinander Werte von 20 bis 30 aufweisen. Entsprechende Verbindungen können beispielsweise durch Umsetzung von Alkyldiolen HO-CHR-CH₂-OH mit Ethylenoxid erhalten werden, wobei im Anschluss eine Umsetzung mit einem Alkylepoxid zum Verschluss der freien OH-Funktionen unter Ausbildung eines Dihydroxyethers erfolgt.

In einer weiteren bevorzugten Ausführungsform ist das nichtionische Tensid ausgewählt aus nichtionischen Tensiden der allgemeinen Formel
R¹-O(CH₂CH₂O)ₓCR³R⁴(OCH₂CH₂)_{y}O-R², in der
- R¹ und R² unabhängig voneinander für einen Alkylrest oder Alkenylrest mit 4 bis 22 Kohlenstoffatomen;
- R³ und R4 unabhängig voneinander für H oder für einen Alkylrest oder Alkenylrest mit 1 bis 18 Kohlenstoffatomen und
- x und y unabhängig voneinander für Werte zwischen 1 und 40 stehen.

Bevorzugt sind hierbei insbesondere Verbindungen der allgemeinen Formel R¹-O(CH₂CH₂O)ₓCR³R⁴(OCH₂CH₂)_{y}O-R², in der R³ und R⁴ für H stehen und die Indices x und y unabhängig voneinander Werte von 1 bis 40, vorzugsweise von 1 bis 15 annehmen.

Besonders bevorzugt sind insbesondere Verbindungen der allgemeinen Formel R¹-O(CH₂CH₂O)ₓCR³R⁴(OCH₂CH₂)_{y}O-R², in der die Reste R¹ und R² unabhängig voneinander gesättigte Alkylreste mit 4 bis 14 Kohlenstoffatome darstellen und die Indices x und y unabhängig voneinander Werte von 1 bis 15 und insbesondere von 1 bis 12 annehmen.

Weiterhin bevorzugt sind solche Verbindungen der allgemeinen Formel R¹-O(CH₂CH₂O)ₓCR³R⁴(OCH₂CH₂)_{y}O-R², in der einer der Reste R¹ und R² verzweigt ist.

Ganz besonders bevorzugt sind Verbindungen der allgemeinen Formel R¹-O(CH₂CH₂O)ₓCR³R⁴(OCH₂CH₂)_{y}O-R², in der die Indices x und y unabhängig voneinander Werte von 8 bis 12 annehmen.

Die angegebenen C-Kettenlängen sowie Ethoxylierungsgrade bzw. Alkoxylierungsgrade der vorgenannten Niotenside stellen statistische Mittelwerte dar, die für ein spezielles Produkt eine ganze oder eine gebrochene Zahl sein können. Aufgrund der Herstellverfahren bestehen Handelsprodukte der genannten Formeln zumeist nicht aus einem individuellen Vertreter, sondern aus Gemischen, wodurch sich sowohl für die C-Kettenlängen als auch für die Ethoxylierungsgrade bzw. Alkoxylierungsgrade Mittelwerte und daraus folgend gebrochene Zahlen ergeben können.

Selbstverständlich können die vorgenannten nichtionischen Tenside nicht nur als Einzelsubstanzen, sondern auch als Tensidgemische aus zwei, drei, vier oder mehr Tensiden eingesetzt werden. Als Tensidgemische werden dabei nicht Mischungen nichtionischer Tenside bezeichnet, die in ihrer Gesamtheit unter eine der oben genannten allgemeinen Formeln fallen, sondern vielmehr solche Mischungen, die zwei, drei, vier oder mehr nichtionische Tenside enthalten, die durch unterschiedliche der vorgenannten allgemeinen Formeln beschrieben werden können.

Durch den Einsatz der zuvor beschriebenen nichtionischen Tenside mit einer freien Hydroxylgruppe an einer der beiden endständigen Alkylreste kann im Vergleich zu herkömmlichen polyalkoxylierten Fettalkoholen ohne freie Hydroxylgruppe die Klarspülleistung und Trocknung deutlich verbessert werden.

Der Gehalt der nichtionischen Tenside, bezogen auf die Summe der Komponenten A und B, beträgt in einer bevorzugten Ausführungsform 0,1 bis 20 Gew.-%, besonders bevorzugt 0,5 bis 15 Gew.-%, insbesondere 2,0 bis 10 Gew.-%.

Weitere Ausführungsformen der vorliegenden Erfindung können als weiteren Bestandteil mindestens ein weiteres anionisches Polymer enthalten. Bevorzugte anionische Polymere sind hierbei die copolymeren Polycarboxylate. Der Gewichtsanteil des anionischen Polymers, bezogen auf die Summe der Komponenten A und B, kann 0,1 bis 20 Gew.-%, vorzugsweise von 0,5 bis 18 Gew.-%, besonders bevorzugt von 1,0 bis 15 Gew.-% und insbesondere von 4 bis 14 Gew.-% betragen.

Ein bevorzugter Gegenstand der vorliegenden Erfindung umfasst als ein weiteres copolymeres anionisches Polymer solche auf, die ausgewählt sind aus der Gruppe der hydrophob modifizierten Polycarboxylate. Diese können in Kombination mit den Sulfopolymeren in maschinellen Geschirrspülmitteln eine Verbesserung der Klarspül- und Trocknungseigenschaften dieser Mittel bei gleichzeitig geringer Belagsbildung erzielen.

Auch kationische bzw. amphotere Polymere können geeignet sein. Zu diesen zählen beispielsweise Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, Polyethylenimin, Copolymere der Acrylsäure mit Dimethyldiallylammoniumchlorid (Merquat® 550/Chemviron), Polyaminopolyamide sowie deren vernetzte wasserlöslichen Polymere, quaternierte Ammoniumsalz-Polymere. Insbesondere bevorzugt werden amphotere Polymere, wie sie in den Offenlegungsschriften WO 2010/107554 und WO 2010/000629 beschrieben werden. Hierbei handelt es sich um Polymere, enthaltend
a) mindestens ein Monomer gemäß der Formel 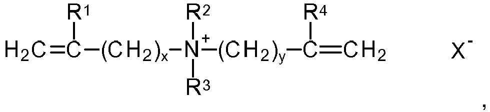 wobei
   R₁ und R₄ unabhängig voneinander für Wasserstoff oder C₁₋₆-Alkyl stehen;
   R₂ und R₃ unabhängig voneinander für C₁₋₆-Alkyl, Hydroxy-C₁₋₆-alkyl oder Amino-C₁₋₆-alkyl stehen;
   m und n unabhängig voneinander für einen Wert von 1 bis 3 stehen;
   X⁻ für ein Gegenion steht;
b) mindestens ein hydrophiles Monomer, das mindestens eine Säure-Gruppe trägt;
c) mindestens ein hydrophiles nichtionisches Monomer.

Als Alkaliträger gelten beispielsweise die Hydroxide, vorzugsweise Alkalimetallhydroxide, die Carbonate, Hydrogencarbonate oder Sesquicarbonate, vorzugsweise Alkalimetallcarbonate bzw. Alkalimetallhydrogencarbonate oder Alkalimetallsesquicarbonate, wobei im Sinne dieser Erfindung bevorzugt die Alkalimetallhydroxide und Alkalicarbonate, insbesondere Natriumhydroxid, Kaliumhydroxide, Natriumcarbonat, Natriumhydrogencarbonat oder Natriumsesquicarbonat eingesetzt werden.

Es hat sich gezeigt, dass sich die Leistung der Reinigungsmittel durch organische Lösungsmittel verbessern lässt. Diese organischen Lösungsmittel stammen beispielsweise aus den Gruppen der Mono-Alkohole, Diole, Triole bzw. Polyole, der Ether, Ester und/oder Amide. Besonders bevorzugt sind dabei organische Lösungsmittel, die wasserlöslich sind, wobei "wasserlösliche" Lösungsmittel im Sinne der vorliegenden Anmeldung Lösungsmittel sind, die bei Raumtemperatur mit Wasser vollständig, d.h. ohne Mischungslücke, mischbar sind.

Organische Lösungsmittel, die in den erfindungsgemäßen Mitteln eingesetzt werden können, stammen vorzugsweise aus der Gruppe ein- oder mehrwertigen Alkohole, Alkanolamine oder Glykolether, sofern sie im angegebenen Konzentrationsbereich mit Wasser mischbar sind. Vorzugsweise werden die Lösungsmittel ausgewählt aus Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykolmethylether, Di-ethylenglykolethylether, Propylenglykolmethyl-, -ethyl- oder - propylether, Dipropylenglykolmethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel.

Als besonders wirkungsvoll im Hinblick auf die Reinigungsleistung und hier wiederum hinsichtlich der Reinigungsleistung an bleichbaren Anschmutzungen, insbesondere an Teeanschmutzungen haben sich die organischen Lösungsmittel aus der Gruppe der organischen Amine und/oder der Alkanolamine.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber den mit den farbstoffhaltigen Mitteln zu behandelnden Substraten wie beispielsweise Textilien, Glas, Keramik oder Kunststoffgeschirr, um diese nicht anzufärben.

Weiterhin können Konservierungsmittel in den Mitteln enthalten sein. Geeignet sind beispielsweise Konservierungsmittel aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-Acetale sowie -Formale, Benzamidine, Isothiazole und deren Derivate wie Isothiazoline und Isothiazolinone, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propynyl-butyl-carbamat, Iod, lodophore und Peroxide. Bevorzugte antimikrobielle Wirkstoffe werden vorzugsweise ausgewählt aus der Gruppe umfassend Ethanol, n-Propanol, i-Propanol, 1,3-Butandiol, Phenoxyethanol, 1,2-Propylenglykol, Glycerin, Undecylensäure, Zitronensäure, Milchsäure, Benzoesäure, Salicylsäure, Thymol, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraazatetradecandiimidamid, antimikrobielle quaternäre oberflächenaktive Verbindungen, Guanidine. Besonders bevorzugte Konservierungsmittel sind jedoch ausgewählt aus der Gruppe umfassend Salicylsäure, quaternäre Tenside, insbesondere Benzalkoniumchlorid und Isothiazole und deren Derivate wie Isothiazoline und Isothiazolinone.

Der pH-Wert bei 20 °C der Komponenten A und B liegt im üblichen Rahmen. Vorzugsweise weist die enzymhaltige Komponente A einen pH-Wert (20°C) zwischen 6 und 9 auf, während die Komponente B einen pH-Wert (20°C) zwischen 9 und 14 aufweist.

### Beispiele

### Beispiel 1: Reinigungsleistung

Je 19,3 g der Komponenten A und B der Produkte 1 (erfindungsgemäß) und 2 (Vergleich) wurden auf ihre Reinigungsleistung nach zwei Wochen Lagerung bei 40 °C im Hauptspülgang eines Geschirrspülers Miele G698SC (Programm 50 °C; Wasser einer Wasserhärte von 21° dH) unter IKW-Standardbedingungen getestet. Die Unterschiede zwischen erfindungsgemäßem Produkt 1 und Vergleichsprodukt 2 werden vor allem an Hackfleisch, Haferflocken und Stärke deutlich.

**Tabelle 1: Zusammensetzungen der Komponenten A in Gew.-%**

| Komponente A | Produkt 1 (erfindungsgemäß) | Produkt 2 (Vergleich) |
|---|---|---|
| Tripolyphosphat | 10,0 | 17,5 |
| Sorbit | 10,05 | 10,05 |
| Ca-lactat | 1,0 | 1,0 |
| Zinkacetat | 0,19 | 0,19 |
| Protease-Präparation | 3,0 | 3,0 |
| Amylase-Präparation | 1,5 | 1,5 |
| Fettalkohol-(EO)₂₂-2-hydroxyalkylether | 4,0 | 4,0 |
| Acrylsäure-Sulfopolymer (Na-Salz) | 5,7 | 0,0 |
| Phosphonat | 0,0 | 1,8 |
| Borsäure | 3,0 | 3,0 |
| Kaliumhydroxid | 3,38 | 3,15 |
| Quervernetzter Acrylsäure-Copolymer-Verdicker | 0,86 | 0,86 |
| Konservierungsmittel | 0,02 | 0,02 |
| Wasser | Ad 100 | Ad 100 |
| pH-Wert (unverdünnt) | 7,5 | 7,5 |

**Tabelle 2: Zusammensetzungen der Komponenten B in Gew.-%**

| Komponente B | Produkt 1 (erfindungsgemäß) | Produkt 2 (Vergleich) |
|---|---|---|
| Tripolyphosphat | 17,5 | 10,0 |
| Amphoteres Polymer | 1,0 | 1,0 |
| Acrylsäure-Sulfopolymer (Na-Salz) | 0,0 | 6,0 |
| Phosphonat | 4,8 | 3,0 |
| Kaliumhydroxid | 3,2 | 3,2 |
| Natriumcarbonat | 10,0 | 10,0 |
| Monoethanolamin | 3,0 | 3,0 |
| Quervernetzter Acrylsäure-Copolymer-Verdicker | 0,92 | 0,88 |
| Wasser | Ad 100 | Ad 100 |
| pH-Wert (unverdünnt) | 11,2 | 11,2 |

**Tabelle 3: Reinigungsergebnisse für verschiedene Anschmutzungen**

| | Milch | Hackfleisch | Eigelb | Haferflocken | Stärkemix |
|---|---|---|---|---|---|
| Produkt 1 | 6,2 | 5,0 | 2,4 | 6,9 | 6,7 |
| Produkt 2 | 6,0 | 2,7 | 1,8 | 1,8 | 4,1 |

### Beispiel 2: Einfluss von Sulfopolymer und Phosphonat auf Enzymstabilität

Es wurden vier verschiedene Komponenten A (A1 ohne Phosphonat und ohne Sulfopolymer; A2 ohne Phosphonat und mit Sulfopolymer; A3 mit Phosphonat und ohne Sulfopolymer; A4 mit Phosphonat und mit Sulfopolymer) aufgemischt und 7 Tage bei 40 °C gelagert. Anschließend wurden die Restaktivitäten der Protease und der Amylase bestimmt. Zum Einsatz kamen eine handelsübliche Protease und eine handelsübliche Amylase. Die Anfangsaktivität wurde jeweils auf 100% gesetzt.

**Tabelle 4: Zusammensetzungen A1 bis A4**

| Inhaltsstoffe | A1 | A2 | A3 | A4 |
|---|---|---|---|---|
| Tripolyphosphat | 10,0 | 10,0 | 10,0 | 10,0 |
| Sorbit | 10,05 | 10,05 | 10,05 | 10,05 |
| Ca-lactat | 1,0 | 1,0 | 1,0 | 1,0 |
| Zinkacetat | 0,19 | 0,19 | 0,19 | 0,19 |
| Protease-Präparation | 3,0 | 3,0 | 3,0 | 3,0 |
| Amylase-Präparation | 1,5 | 1,5 | 1,5 | 1,5 |
| Fettalkohol-(EO)₂₂-2-hydroxyalkylether | 4,0 | 4,0 | 4,0 | 4,0 |
| Acrylsäure-Sulfopolymer (Na-Salz) | --- | 5,7 | --- | 5,7 |
| Phosphonat | --- | --- | 1,8 | 1,8 |
| Borsäure | 3,0 | 3,0 | 3,0 | 3,0 |
| Kaliumhydroxid | 1,89 | 3,4 | 2,9 | 4,7 |
| Quervernetzter Acrylsäure-Copolymer-Verdicker | 0,86 | 0,86 | 0,86 | 0,86 |
| Konservierungsmittel | 0,02 | 0,02 | 0,02 | 0,02 |
| Wasser | Ad 100 | Ad 100 | Ad 100 | Ad 100 |

Die jeweilige proteolytische Restaktivität wurde über die Freisetzung des Chromophors para-Nitroanilin (pNA) aus dem Substrat bestimmt. Bei dem Substrat handelte es sich um suc-L-Ala-L-Ala-L-Pro-L-Phe-p-Nitroanilid (suc-AAPF-pNA). Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979).

Die Restaktivität der eingesetzten Amylase wurde mittels der Methode bestimmt, die in M. Lever, Carbohydrate Determination with 4-hydroxybenzoic acid hydrazide (PAHBAH): Effect of Bismuth on the Reaction, Anal. Biochem., 1977, 81, Seiten 21 bis 27 beschrieben ist.

**Tabelle 5: Restaktivität der Protease**

| Rezeptur | Protease |
|---|---|
| A1 | 36% |
| A2 | 48% |
| A4 | 74% |

Im Falle der Protease ist der stabilisierende Einfluss des Sulfopolymers (A2 und A4) gegenüber einer Rezeptur ohne Sulfopolymer deutlich zu erkennen. Die besten Werte liefert die Kombination von Phosphonat und Sulfopolymer (A4).

**Tabelle 6: Restaktivität der Amylase**

| Rezeptur | Amylase |
|---|---|
| A1 | 38% |
| A2 | 65% |
| A3 | 36% |
| A4 | 62% |

Auch im Falle der Amylase zeigen die Rezepturen mit Sulfopolymer (A2 und A4) die besten Stabilitäten. Das Vorhandensein von Phosphonat bewirkt eine leichte Destabilisierung (A2 gegenüber A3 und A4 gegenüber A3).

## Patentansprüche

1. Verwendung von Sulfopolymer in wässerigen Systemen, insbesondere in flüssigen maschinellen Geschirrspülmitteln oder Komponenten hierzu, zur Stabilisierung von Proteasen und/oder Amylasen.

2. Verwendung gemäß Anspruch 1, **dadurch gekennzeichnet, dass** 0,1 bis 15 Gew.-% Sulfopolymer und 0,1 bis 10 Gew.-% einer Enzympräparation, enthaltend Amylase- und/oder Protease, verwendet werden, wobei die Konzentrationen auf das wässerige System, insbesondere auf das flüssige maschinelle Geschirrspülmittel oder eine Komponente hierfür bezogen sind.

3. Kombinationsprodukt, umfassend ein Verpackungsmittel und mindestens zwei in diesem Verpackungsmittel befindliche voneinander getrennte, wässerige Wasch- oder Reinigungskomponenten A und B, wobei A und B enthalten
A:
- 10 bis 74,9 Gew.-% Gerüststoff(e);
- 0,1 bis 10 Gew.-% Enzympräparation;
- 24,9 bis 89,8 Gew.-% Wasser; und
B:
- 10 bis 75 Gew.-% Gerüststoff(e);
- weniger als 0,1 Gew.-% Enzympräparation;
- 25 bis 90 Gew.-% Wasser;
**dadurch gekennzeichnet, dass** die Komponente A zusätzlich mindestens ein Sulfopolymer, vorzugsweise in Mengen von 0,01 bis 15 Gew.-%, bezogen auf die Komponente A, und die Enzympräparation der Komponente A Protease und/oder Amylase enthält.

4. Kombinationsprodukt gemäß Anspruch 3, **dadurch gekennzeichnet, dass** die Komponente A 1 bis 15 Gew.-% Sulfopolymer und vorzugsweise 1,5 bis 10 Gew.-% Sulfopolymer, jeweils bezogen auf die Komponente A, enthält.

5. Kombinationsprodukt gemäß einem der Ansprüche 3 oder 4, **dadurch gekennzeichnet, dass** nur die Komponente A Sulfopolymer enthält oder die Komponenten A und B Sulfopolymer enthalten, wobei der Gewichtsanteil des Sulfopolymers in der Komponente A, bezogen auf die Komponente A, höher ist als der Gewichtsanteil des Sulfopolymers in der Komponente B, bezogen auf die Komponente B.

6. Kombinationsprodukt gemäß einem der Ansprüche 3 bis 5, **dadurch gekennzeichnet, dass** die Komponente A eine Protease gemäß SEQ ID NO. 1 enthält.

7. Kombinationsprodukt gemäß einem der Ansprüche 3 bis 6, **dadurch gekennzeichnet, dass** die Komponente A eine Amylase gemäß SEQ ID NO. 2 enthält.

8. Kombinationsprodukt gemäß einem der Ansprüche 3 bis 7, **dadurch gekennzeichnet, dass** das Verpackungsmittel einen wasserunlöslichen Zwei- oder Mehrkammerbehälter darstellt, wobei vorzugsweise jede der Aufnahmekammern des Verpackungsmittels mit einem Ausguss versehen ist.

9. Kombinationsprodukt gemäß einem der Ansprüche 3 bis 7, **dadurch gekennzeichnet, dass** das Verpackungsmittel einen wasserlöslichen Zwei- oder Mehrkammerbeutel darstellt.

10. Automatisches Wasch- oder Reinigungsverfahren, insbesondere maschinelles Geschirrspülverfahren, **dadurch gekennzeichnet, dass**
entweder unter Einsatz eines Kombinationsprodukts gemäß einem der Ansprüche 3 bis 8 die Wasch- oder Reinigungskomponenten A und B aus einem wasserunlöslichen Verpackungsmittel in eine in der Maschine vorgesehene oder in eine separate Dosiervorrichtung oder direkt in die Maschine dosiert werden
oder unter Einsatz eines Kombinationsprodukts gemäß einem der Ansprüche 3 bis 7 und 9 das wasserlösliche Verpackungsmittel in eine in der Maschine vorgesehene oder in eine separate Dosiervorrichtung dosiert oder direkt in den Innenraum der Maschine gelegt
und
anschließend der Wasch- oder Reinigungsvorgang in Gang gesetzt wird.
